(19) Europäisches Patentamt
European Patent Office
Office européen des brevets

(11) Veröffentlichungsnummer: **0 173 208**
**B1**

(12) # EUROPÄISCHE PATENTSCHRIFT

(45) Veröffentlichungstag der Patentschrift: **01.06.88**

(21) Anmeldenummer: **85110359.8**

(22) Anmeldetag: **19.08.85**

(51) Int. Cl.[4]: **C 07 D 401/06**, C 07 D 215/18, C 07 D 215/08, C 07 D 215/12, C 07 D 215/24, C 07 D 215/36, C 07 D 221/10, A 01 N 47/38 // (C07D401/06, 215:18, 233:61)

(54) **Tetrahydrochinolin-1-ylcarbonylimidazol-Derivate.**

(30) Priorität: **30.08.84 JP 179399/84**

(43) Veröffentlichungstag der Anmeldung: **05.03.86 Patentblatt 86/10**

(45) Bekanntmachung des Hinweises auf die Patenterteilung: **01.06.88 Patentblatt 88/22**

(84) Benannte Vertragsstaaten: **AT BE CH DE FR GB IT LI NL**

(73) Patentinhaber: **NIHON TOKUSHU NOYAKU SEIZO K.K., No.4, 2-chome, Nihonbashi Honcho Chuo-ku, Tokyo 103 (JP)**

(72) Erfinder: **Kurahashi, Yoshio, 47-15, Oya-machi Hachioji-shi, Tokyo (JP)**
Erfinder: **Shiokawa, Kozo, 210-6, Shukugawara Tama-ku, Kawasaki-shi Kanagawa-ken (JP)**
Erfinder: **Goto, Toshio, 3454-21, Honmachida, Machida-shi Tokyo (JP)**
Erfinder: **Kagabu, Shinzo, 432-131-105, Terada-machi, Hachioji shi Tokyo (JP)**
Erfinder: **Kamochi, Atsumi, 2-24-10 Higashi-Toyoda, Hino-shi Tokyo (JP)**
Erfinder: **Moriya, Koichi, 39-15, Namiki-cho, Hachioji-shi Tokyo (JP)**
Erfinder: **Hayakawa, Hidenori, 3-3-19, Midori-cho, Musashino-shi Tokyo (JP)**

(74) Vertreter: **Schumacher, Günter, Dr. et al, c/o Bayer AG Konzernverwaltung RP Patentabteilung, D-5090 Leverkusen 1 Bayerwerk (DE)**

(56) Entgegenhaltungen:
**DE - A - 1 905 606**
**DE - A - 2 260 025**
**DE - A - 3 129 718**
**GB - A - 1 487 283**

**CHEMICAL ABSTRACTS, Band 73, Nr. 13, 28. September 1970, Columbus, Ohio, USA PESSON, MARCEL; ANTOINE, MICHEL "1,2,4-Triazoles. V. N, N-Dialkylamides derived from 1,2,4-triazole-5-carboxylic acids" Seite 340, Spalte 1, Zusammenfassung-Nr. 66 517n**
**CHEMICAL ABSTRACTS, Band 92, Nr. 7, 18. Februar 1980, Columbus, Ohio, USA IKURA, KATSUYATA; KATSUURA, KIYOSHI; MIZUNO, MASAMI; NISHIBE,**

(56) Entgegenhaltungen: (Fortsetzung)
**TADAYUKI "Imidazolium halides" Seite 676, Spalte 1, Zusammenfassung-Nr. 58 776y**
**CHEMICAL ABSTRACTS, Band 99, Nr. 13, 26. September 1983, Columbus, Ohio, USA NIHON NOHYAKU CO., LTD. "Fungicidal acetamides" Seite 587, Spalte 2, Zusammenfassung-Nr. 105 253g**
**CHEMICAL ABSTRACTS, Band 92, Nr. 19, 12. Mai 1980, Columbus, Ohio, USA WALTER, WOLFGANG; RADKE, MATTHIAS "Reactions of azoles with inorganic acid chlorides" Seite 597, Spalte 2, Zusammenfassung-Nr. 163 905n**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue Tetrahydrochinolin-1-ylcarbonylimidazol-Derivate, Verfahren zu ihrer Herstellung, ihre Verwendung als Herbizide oder Fungizide für Landwirtschaft und Gartenbau sowie neue Zwischenprodukte für diese und Verfahren zu deren Herstellung.

Es wurde bereits offenbart, dass bestimmte tertiäre Carbamoyltriazole und bestimmte Imidazolderivate insektizide Aktivität besitzen (vgl. die US-PS 3 308 131 und DE-OS 2 260 025). So kann beispielsweise 1-(4-Methylpiperidinocarbonyl)-1,2,4-triazol zur Tötung von Schadinsekten eingesetzt werden. Weiterhin sind bestimmte 1-Acyl-hydrochinoline als Herbizide bekannt (vgl. DE-OS 1 905 606).

Nunmehr wurden neue Tetrahydrochinolin-1-ylcarbonylimidazol-Derivate der Formel (I)

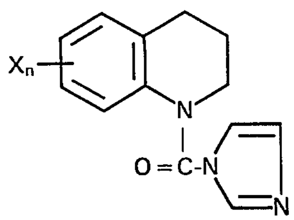

I

gefunden, in der

X ein Halogen-Atom, eine $C_1$- bis $C_4$-Alkyl-Gruppe, eine Nitro-Gruppe, eine Cyano-Gruppe, eine Trifluoromethyl-Gruppe, eine $C_1$- bis $C_4$-Alkoxy-Gruppe, die durch ein Halogen-Atom substituiert sein kann, eine $C_1$- bis $C_4$-Alkylthio-Gruppe, die durch ein Halogen-Atom substituiert sein kann, eine $C_1$- bis $C_4$-Alkylsulfonyl-Gruppe, eine Phenyl-Gruppe, eine Phenylthio-Gruppe, die durch ein Halogen-Atom substituiert sein kann, oder eine Phenoxy-Gruppe, die durch wenigstens einen aus der aus einem Halogen-Atom, einer Trifluormethyl-Gruppe, einer Nitro-Gruppe und $C_1$- bis $C_4$-Alkyl-Gruppen bestehenden Klasse ausgewählten Substituenten substituiert sein kann, bezeichnet und

n 1, 2 oder 3 bezeichnet, und, sofern n 2 oder 3 ist, die zwei oder drei durch X bezeichneten Substituenten gleich oder verschieden sein können und zwei Substituenten X zusammen mit den Kohlenstoff-Atomen in den Positionen 7 und 8 des Tetrahydrochinolin-Rings einen gesättigten oder ungesättigten Kohlenwasserstoff-Ring mit 5 bis 6 Kohlenstoff-Atomen bilden können,

mit der Massgabe, dass, wenn n 1 ist, X kein Chlor-Atom ist, und wenn n 2 oder 3 ist, alle X nicht gleichzeitig Chlor-Atome sind. Diese Verbindungen sind Gegenstand der älteren Anmeldung EP-A-158 954.

Tetrahydrochinolin-1-ylcarbonylimidazol-Derivate der Formel (I) werden erhalten, wenn

(a) Verbindungen der Formel (II)

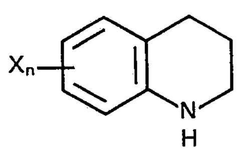

II

in der

X und n die im Vorstehenden angegebenen Bedeutungen haben, mit N,N'-carbonyldiimidazol der Formel

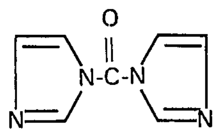

gegebenenfalls in Gegenwart inerter Lösungsmittel, umgesetzt werden oder

(b) Verbindung der Formel (III)

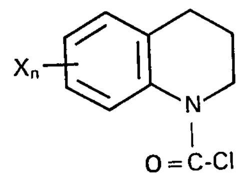

III

in der

X und n die im Vorstehenden angegebenen Bedeutungen haben, mit Imidazol der Formel

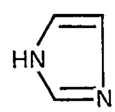

gegebenenfalls in Gegenwart von Säureacceptoren, umgesetzt werden.

Die neuen Tetrahydrochinolin-1-ylcarbonylimidazol-Derivate zeigen starke herbizide und in Landwirtschaft und Gartenbau nutzbare fungizide Eigenschaften.

Ebenfalls gefunden wurden neue Tetrahydrochinolin-1-ylcarbonylchloride der Formel (III), die Zwischenprodukte bei der Herstellung der Verbindungen der Formel (I) sind. Tetrahydrochinolin-1-ylcarbonylchloride der Formel (III) werden erhalten, wenn die Verbindungen der Formel (II) mit Phosgen oder Chlorameisensäuretrichlormethylester umgesetzt werden.

Überraschenderweise zeigen die Tetrahydrochinolin-1-ylcarbonylimidazol-Derivate gemäss der vorliegenden Erfindung wesentlich stärkere herbizide oder fungizide Wirkung als aus dem Stand der Technik bekannte Verbindungen und insbesondere eine ausgezeichnete Bekämpfungswirkung gegen Unkräuter in Reisfeldern und gleichzeitig eine günstige Verträglichkeit mit Reis.

Unter den Tetrahydrochinolin-1-ylcarbonylimidazol-Derivaten der Formel (I) gemäss der vorliegenden Erfindung sind diejenigen bevorzugt, in denen

X Fluor, Chlor, Brom, Alkyl mit 1 bis 4 Kohlenstoff-Atomen, Trifluoromethyl oder Alkoxy mit 1 bis 4 Kohlenstoff-Atomen, das durch Fluor und/oder Chlor substituiert sein kann, bezeichnet und

n 1 oder 2 bezeichnet, und, wenn n 2 ist, die beiden X identisch oder unterschiedlich sein können und zwei Substituenten X zusammen mit den Kohlenstoff-Atomen in den Positionen 7 und 8 des Tetrahydrochinolin-Rings einen gesättigten oder ungesättigten Kohlenwasserstoff-Ring mit 5 bis 6 Kohlenstoff-Atomen bilden können,
mit der Massgabe, dass, wenn n 1 ist, X kein Chlor ist, und wenn n 2 ist, beide X nicht gleichzeitig Chlor sind.

Besonders bevorzugte Verbindungen der Formel (I) sind diejenigen, in denen

X Fluor, Chlor, Methyl, Ethyl, n-Propyl, Isopropyl, Trifluormethyl oder Methoxy, Ethoxy, n-Propoxy oder Isopropoxy, die durch Fluor und/oder Chlor substituiert sein können, bezeichnet und

n 2 bezeichnet und die beiden X identisch oder unterschiedlich sein können und die zwei Substituenten X zusammen mit den Kohlenstoff-Atomen in den Positionen 7 und 8 des Tetrahydrochinolin-Rings einen gesättigten oder ungesättigten Kohlenwasserstoff-Ring mit 5 bis 6 Kohlenstoff-Atomen bilden können,
mit der Massgabe, dass, wenn n 2 ist, beide X nicht gleichzeitig Chlor sind.

Speziell erwähnt seien die folgenden Verbindungen:

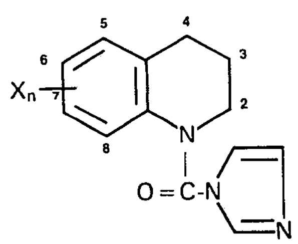

| $X_n$ |
|---|
| 7-Cl, 8-$CH_3$ |
| 7,8-$(CH_3)_2$ |
| 7-$CH_3$, 8-Cl |
| 7,8-$F_2$ |
| 7-Cl, 8-$CF_3$ |
| 7-F, 8-Cl |
| 7,8-$(OCH_3)_2$ |

Formel:

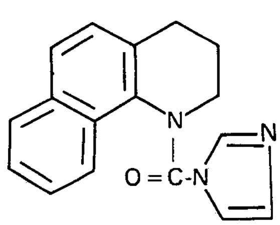

7-F, 8-$CH_3$

Formel:

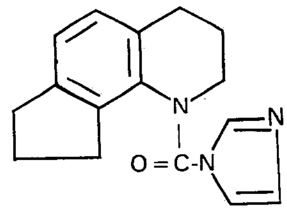

Formel:

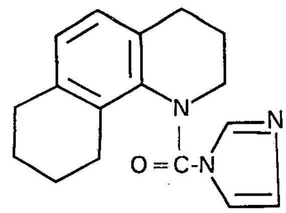

Wenn beispielsweise 7-Chloro-8-methyl-1,2,3,4-tetrahydrochinolin und N,N'-Carbonyldiimidazol als Ausgangsstoffe eingesetzt werden, kann der Reaktionsablauf durch die nachstehende Gleichung dargestellt werden:

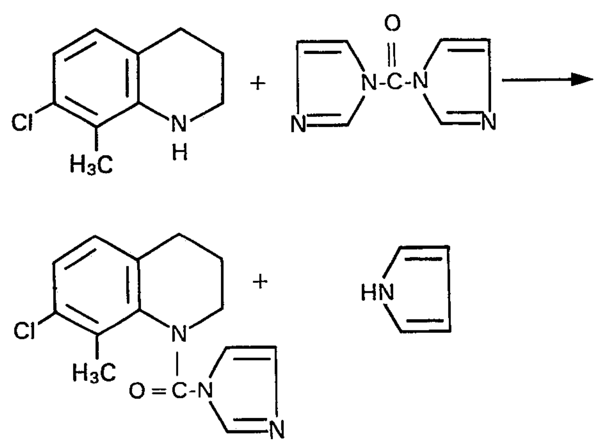

Wenn beispielsweise 7,8-Dimethyl-1(H),2,3,4-tetrahydrochinolin-1-yl-carbonylchlorid und Imidazol als Ausgangsstoffe eingesetzt werden, kann der Reaktionsablauf durch die nachstehende Gleichung dargestellt werden:

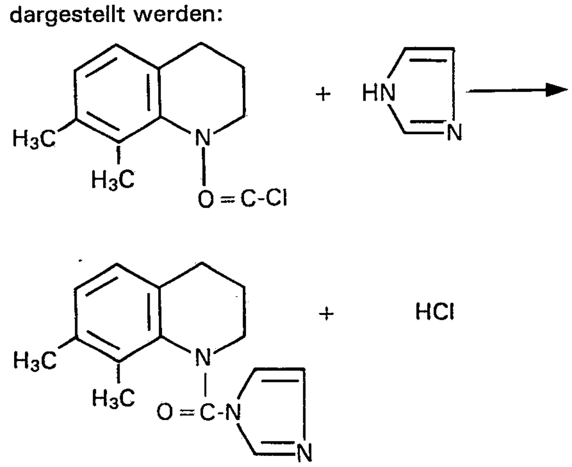

Die Formel (II) liefert eine allgemeine Definition für die Verbindungen, die als Ausgangsstoffe in der Reaktionsvariante (a) gemäss der vorliegenden Erfindung benötigt werden.

In der Formel (II) haben X und n vorzugsweise die bereits im Vorstehenden angegebenen Bedeutungen.

Die gemäss der vorliegenden Erfindung einsetzbaren Verbindungen sind zum Teil bereits bekannt.

Als Beispiele seien erwähnt:

6-Methyl-1,2,3,4-tetrahydrochinolin,
6-Ethyl-1,2,3,4-tetrahydrochinolin,
6-Isopropyl-1,2,3,4-tetrahydrochinolin,
6-tert-Butyl-1,2,3,4-tetrahydrochinolin,
7-Methyl-1,2,3,4-tetrahydrochinolin,
8-Methyl-1,2,3,4-tetrahydrochinolin,
8-Ethyl-1,2,3,4-tetrahydrochinolin,
8-Propyl-1,2,3,4-tetrahydrochinolin,
8-Fluoro-1,2,3,4-tetrahydrochinolin,
8-Bromo-1,2,3,4-tetrahydrochinolin,
5-Fluoro-1,2,3,4-tetrahydrochinolin,
6-Fluoro-1,2,3,4-tetrahydrochinolin,
7-Fluoro-1,2,3,4-tetrahydrochinolin,
6-Bromo-1,2,3,4-tetrahydrochinolin,
7-Bromo-1,2,3,4-tetrahydrochinolin,
8-Trifluoromethyl-1,2,3,4-tetrahydrochinolin,
8-Methoxy-1,2,3,4-tetrahydrochinolin,
6-Methoxy-1,2,3,4-tetrahydrochinolin,
6-Isopropoxy-1,2,3,4-tetrahydrochinolin,
7-Methoxy-1,2,3,4-tetrahydrochinolin,
7-Isopropoxy-1,2,3,4-tetrahydrochinolin,
8-Ethoxy-1,2,3,4-tetrahydrochinolin,
6,8-Dimethyl-1,2,3,4-tetrahydrochinolin,
7,8-Dimethyl-1,2,3,4-tetrahydrochinolin,
6,7-Dimethyl-1,2,3,4-tetrahydrochinolin,
6-Chloro-8-methyl-1,2,3,4-tetrahydrochinolin,
8-Chloro-6-methyl-1,2,3,4-tetrahydrochinolin,
7-Chloro-8-methyl-1,2,3,4-tetrahydrochinolin,
6-Chloro-8-trifluoromethyl-1,2,3,4-tetrahydrochinolin,
8-Chloro-5-methyl-1,2,3,4-tetrahydrochinolin,
7-Chloro-6-methyl-1,2,3,4-tetrahydrochinolin,
8-Chloro-7-methyl-1,2,3,4-tetrahydrochinolin,
5-Chloro-8-methyl-1,2,3,4-tetrahydrochinolin,
6-Bromo-8-methyl-1,2,3,4-tetrahydrochinolin,
5-Chloro-8-methoxy-1,2,3,4-tetrahydrochinolin,
6,8-Difluoro-1,2,3,4-tetrahydrochinolin,
7,8-Difluoro-1,2,3,4-tetrahydrochinolin,
6-Trifluoromethyl-1,2,3,4-tetrahydrochinolin,
7-Trifluoromethyl-1,2,3,4-tetrahydrochinolin,
5-Chloro-8-trifluoromethyl-1,2,3,4-tetrahydrochinolin,
6-Chloro-7-trifluoromethyl-1,2,3,4-tetrahydrochinolin,
7-Chloro-8-trifluoromethyl-1,2,3,4-tetrahydrochinolin,
6,7-Difluoro-1,2,3,4-tetrahydrochinolin,
6,8-Dibromo-1,2,3,4-tetrahydrochinolin,
7,8-Dibromo-1,2,3,4-tetrahydrochinolin,
8-Chloro-6-fluoro-1,2,3,4-tetrahydrochinolin,
6-Chloro-8-fluoro-1,2,3,4-tetrahydrochinolin,
8-Chloro-7-fluoro-1,2,3,4-tetrahydrochinolin,
6-Brom-8-chloro-1,2,3,4-tetrahydrochinolin,
6,7-Dimethoxy-1,2,3,4-tetrahydrochinolin,
7,8-Dimethoxy-1,2,3,4-tetrahydrochinolin,
6-Trifluoromethoxy-1,2,3,4-tetrahydrochinolin,
8-Trifluoromethoxy-1,2,3,4-tetrahydrochinolin,
8-Difluoromethoxy-1,2,3,4-tetrahydrochinolin,
Benzo[h]-1,2,3,4-tetrahydrochinolin,
8-Isopropyl-1,2,3,4-tetrahydrochinolin,
8-tert-Butyl-1,2,3,4-tetrahydrochinolin,
8-Isopropoxy-1,2,3,4-tetrahydrochinolin,
7-Fluoro-8-methyl-1,2,3,4-tetrahydrochinolin,
7,8-Trimethylen-1,2,3,4-tetrahydrochinolin, und
7,8-Tetramethylen-1,2,3,4-tetrahydrochinolin.

Einige der hiervor erwähnten Ausgangsstoffe sind neu. Beispielsweise sind 7-Chloro-8-methyl-1,2,-3,4-tetrahydrochinolin und 8-Chloro-7-methyl-1,2,3,4-tetrahydrochinolin neue Verbindungen. Ihre Herstellung ist ausführlicher in einem nachfolgenden Beispiel beschrieben.

Im allgemeinen können die Vorprodukte für die Verbindungen durch Reduktion der entsprechenden Chinoline hergestellt werden, die bekannte Verbindungen sind (vgl. Chemical Abstracts 54, 22650b, und Chemical Abstracts 41, 5517b).

Andere der hiervon erwähnten Ausgangsstoffe sind bereits bekannt. Beispielsweise sind 7,8-Dimethyl-1,2,3,4-tetrahydrochinolin (vgl. Chemical Abstracts 83, 206079), Benzo[h]-1,2,3,4-tetrahydrochinolin [vgl. Journal of Organic Chemistry 40, S. 2729 (1975)] und darüber hinaus in der 8-Stellung substituierte 1,2,3,4-Tetrahydrochinoline (vgl. Journal of the Chemical Society Perkin I, 1980, S. 1933-1938) bereits bekannt. N,N'-Carbonyldiimidazol ist bereits bekannt (vgl. Beilstein, 23 II, S. 35).

Die Formel (III) liefert eine allgemeine Definition für die Verbindungen, die als Ausgangsstoffe in der Reaktionsvariante (b) gemäss der vorliegenden Erfindung benötigt werden.

In der Formel (III) haben X und n vorzugsweise die bereits im Vorstehenden angegebenen Bedeutungen.

Die folgenden Verbindungen seien als Beispiele für Verbindungen der Formel (III) erwähnt:

6-Methyl-1(H),2,3,4-tetrahydrochinolin-1-ylcarbonylchlorid,
6-Ethyl-1(H),2,3,4-tetrahydrochinolin-1-ylcarbonylchlorid,
6-Isopropyl-1(H),2,3,4-tetrahydrochinolin-1-ylcarbonylchlorid,
6-tert-Butyl-1(H),2,3,4-tetrahydrochinolin-1-ylcarbonylchlorid,
7-Methyl-1(H),2,3,4-tetrahydrochinolin-1-ylcarbonylchlorid,
8-Methyl-1(H),2,3,4-tetrahydrochinolin-1-ylcarbonylchlorid,
8-Ethyl-1(H),2,3,4-tetrahydrochinolin-1-ylcarbonylchlorid,
8-Propyl-1(H),2,3,4-tetrahydrochinolin-1-ylcarbonylchlorid,
8-Fluoro-1(H),2,3,4-tetrahydrochinolin-1-ylcarbonylchlorid,
8-Bromo-1(H),2,3,4-tetrahydrochinolin-1-ylcarbonylchlorid,
5-Fluoro-1(H),2,3,4-tetrahydrochinolin-1-ylcarbonylchlorid,

6-Fluoro-1(H),2,3,4-tetrahydrochinolin-1-ylcarbonylchlorid,
7-Fluoro-1(H),2,3,4-tetrahydrochinolin-1-ylcarbonylchlorid,
6-Bromo-1(H),2,3,4-tetrahydrochinolin-1-ylcarbonylchlorid,
7-Bromo-1(H),2,3,4-tetrahydrochinolin-1-ylcarbonylchlorid,
8-Trifluoromethyl-1(H),2,3,4-tetrahydrochinolin-1-ylcarbonylchlorid,
8-Methoxy-1(H),2,3,4-tetrahydrochinolin-1-ylcarbonylchlorid,
6-Methoxy-1(H),2,3,4-tetrahydrochinolin-1-ylcarbonylchlorid,
6-Isopropoxy-1(H),2,3,4-tetrahydrochinolin-1-ylcarbonylchlorid,
7-Methoxy-1(H),2,3,4-tetrahydrochinolin-1-ylcarbonylchlorid,
7-Isopropoxy-1(H),2,3,4-tetrahydrochinolin-1-ylcarbonylchlorid,
8-Ethoxy-1(H),2,3,4-tetrahydrochinolin-1-ylcarbonylchlorid,
6,8-Dimethyl-1(H),2,3,4-tetrahydrochinolin-1-ylcarbonylchlorid,
7,8-Dimethyl-1(H),2,3,4-tetrahydrochinolin-1-ylcarbonylchlorid,
6,7-Dimethyl-1(H),2,3,4-tetrahydrochinolin-1-ylcarbonylchlorid,
6-Chloro-8-methyl-1H),2,3,4-tetrahydrochinolin-1-ylcarbonylchlorid,
8-Chloro-6-methyl-1(H),2,3,4-tetrahydrochinolin-1-ylcarbonylchlorid,
7-Chloro-8-methyl-1(H),2,3,4-tetrahydrochinolin-1-ylcarbonylchlorid,
6-Chloro-8-trifluoromethyl-1(H),2,3,4-tetrahydrochinolin-1-ylcarbonylchlorid,
8-Chloro-5-methyl-1(H),2,3,4-tetrahydrochinolin-1-ylcarbonylchlorid,
7-Chloro-6-methyl-1(H),2,3,4-tetrahydrochinolin-1-ylcarbonylchlorid,
8-Chloro-7-methyl-1(H),2,3,4-tetrahydrochinolin-1-ylcarbonylchlorid,
5-Chloro-8-methyl-1(H),2,3,4-tetrahydrochinolin-1-ylcarbonylchlorid,
6-Bromo-8-methyl-1(H),2,3,4-tetrahydrochinolin-1-ylcarbonylchlorid,
5-Chloro-8-methoxy-1(H),2,3,4-tetrahydrochinolin-1-ylcarbonylchlorid,
6,8-Difluoro-1(H),2,3,4-tetrahydrochinolin-1-ylcarbonylchlorid,
7,8-Difluoro-1(H),2,3,4-tetrahydrochinolin-1-ylcarbonylchlorid,
6-Trifluoromethyl-1(H),2,3,4-tetrahydrochinolin-1-ylcarbonylchlorid,
7-Trifluoromethyl-1(H),2,3,4-tetrahydrochinolin-1-ylcarbonylchlorid,
5-Chloro-8-trifluoromethyl-1(H),2,3,4-tetrahydrochinolin-1-ylcarbonylchlorid,
6-Chloro-7-trifluoromethyl-1(H),2,3,4-tetrahydrochinolin-1-ylcarbonylchlorid,
7-Chloro-8-trifluoromethyl-1(H),2,3,4-tetrahydrochinolin-1-ylcarbonylchlorid,
6,7-Difluoro-1(H),2,3,4-tetrahydrochinolin-1-ylcarbonylchlorid,
6,8-Dibromo-1(H),2,3,4-tetrahydrochinolin-1-ylcarbonylchlorid,
7,8-Dibromo-1(H),2,3,4-tetrahydrochinolin-1-ylcarbonylchlorid,
8-Chloro-6-fluoro-1(H),2,3,4-tetrahydrochinolin-1-ylcarbonylchlorid,
6-Chloro-8-fluoro-1(H),2,3,4-tetrahydrochinolin-1-ylcarbonylchlorid,
8-Chloro-7-fluoro-1(H),2,3,4-tetrahydrochinolin-1-ylcarbonylchlorid,
6-Bromo-8-fluoro-1(H),2,3,4-tetrahydrochinolin-1-ylcarbonylchlorid,
6,7-Dimethoxy-1(H),2,3,4-tetrahydrochinolin-1-ylcarbonylchlorid,
7,8-Dimethoxy-1(H),2,3,4-tetrahydrochinolin-1-ylcarbonylchlorid,
6-Trifluoromethoxy-1(H),2,3,4-tetrahydrochinolin-1-ylcarbonylchlorid,
8-Trifluoromethoxy-1(H),2,3,4-tetrahydrochinolin-1-ylcarbonylchlorid,
8-Difluoromethoxy-1(H),2,3,4-tetrahydrochinolin-1-ylcarbonylchlorid,
Benzo[h]-1(H),2,3,4-tetrahydrochinolin-1-ylcarbonylchlorid,
8-Isopropyl-1(H),2,3,4-tetrahydrochinolin-1-ylcarbonylchlorid,
8-tert-Butyl-1(H),2,3,4-tetrahydrochinolin-1-ylcarbonylchlorid,
8-Isopropoxy-1(H),2,3,4-tetrahydrochinolin-1-ylcarbonylchlorid,
7-Fluoro-8-methyl-1(H),2,3,4-tetrahydrochinolin-1-ylcarbonylchlorid,
7,8-Trimethylen-1(H),2,3,4-tetrahydrochinolin-1-ylcarbonylchlorid und,
7,8-Tetramethylen-1(H),2,3,4-tetrahydrochinolin-1-ylcarbonylchlorid.

Die hiervor erwähnten Verbindungen der Formel (III) sind neu. Ihre Herstellung wird im Folgenden ausführlicher beschrieben.

Wenn beispielsweise 8-Chloro-6-methyl-1,2,3,4-tetrahydrochinolin und Chlorameisensäuretrichloromethylester als Ausgangsstoffe eingesetzt werden, kann der Reaktionsablauf durch die nachstehende Gleichung dargestellt werden:

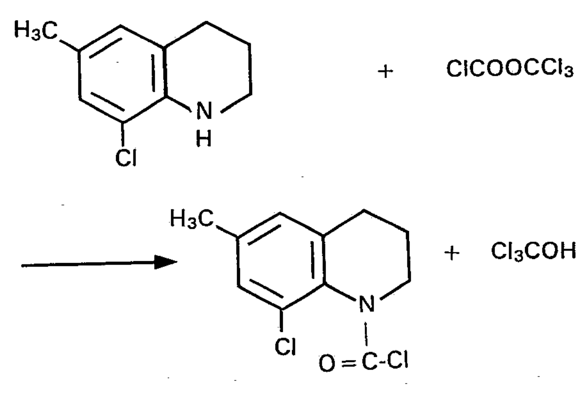


Die Formel (II) gibt eine allgemeine Definition derjenigen Verbindungen, die als Ausgangsstoffe in der Reaktion zur Herstellung der Verbindungen der Formel (III) benötigt werden.

Die Beispiele der Formel (II) können diejenigen der vorstehenden Beschreibung sein.

Die Reaktionsvariante (a) gemäss der vorliegenden Erfindung wird vorzugsweise in Gegenwart eines Lösungsmittels oder Verdünnungsmittels durchgeführt.

Beispiele für solche Lösungsmittel oder Verdünnungsmittel umfassen Wasser; aliphatische, alicyclische und aromatische Kohlenwasserstoffe (die gegebenenfalls chloriert sein können), wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol, Ether wie Diethylether, Methylethylether, Di-isopropylether, Dibutylether, Propylenoxid, Dioxan und Tetrahydrofuran, Ketone wie Aceton, Methylethylketon, Methylisopropylketon und Ethylisobutylketon, Nitrile wie Acetonitril, Propionitril und Acrylnitril, Alkohole wie Methanol, Ethanol, Isopropanol, Butanol und Ethylenglycol, Ester wie Ethylacetat und Amylacetat, Säureamide wie Dimethylformamid und Dimethylacetamid, Sulfone und Sulfoxide wie Dimethylsulfoxid und Sulfolan sowie Basen wie Pyridin.

Die Reaktionsvariante (a) kann innerhalb eines breiten Temperaturbereichs durchgeführt werden. Im allgemeinen kann sie bei einer Temperatur zwischen −20°C und dem Siedepunkt der Mischung, vorzugsweise bei einer Temperatur zwischen 0°C und 100°C, durchgeführt werden. Vorzugsweise wird die Reaktionsvariante (a) unter Umgebungsdruck durchgeführt, jedoch ist es ebenfalls möglich, bei erhöhtem oder vermindertem Druck zu arbeiten.

Bei der Durchführung der Reaktionsvariante (b) werden zur Gewinnung der Endprodukte mit hoher Reinheit in hoher Ausbeute vorzugsweise die gleichen Lösungsmittel oder Verdünnungsmittel verwendet, wie sie im Vorstehenden für die Reaktionsvariante (a) beschrieben wurden.

In gleicher Weise wird die Reaktionsvariante (b) vorzugswseise in Gegenwart eines Säureacceptors durchgeführt.

Beispiele für den Säureacceptor umfassen die Hydroxide, Carbonate, Hydrogencarbonate und Alkoholate von Alkalimetallen sowie tertiäre Amine wie Triethylamin, Diethylanilin und Pyridin, die sämtlich allgemein verwendet werden.

Die Reaktionsvariante (b) kann innerhalb eines breiten Temperaturbereichs durchgeführt werden, beispielsweise bei einer Temperatur zwischen −20°C und dem Siedepunkt der Mischung, vorzugsweise bei einer Temperatur zwischen 0°C und 100°C. Die Reaktionsvariante (b) wird unter Umgebungsdruck durchgeführt, jedoch ist es ebenfalls möglich, bei erhöhtem oder vermindertem Druck zu arbeiten.

Die aktiven Verbindungen der Formel (I) gemäss der vorliegenden Erfindung zeigen herausragende selektive herbizide Aktivität und fungizide Aktivität.

Da die durch die allgemeine Formel (I) dargestellten Verbindungen gemäss der vorliegenden Erfindung gekennzeichnet sind durch niedrige Toxizität gegenüber warmblütigen Tieren und gute Selektivität für Kulturpflanzen, nämlich die Freiheit von Phytotoxizität gegen Kulturpflanzen in den üblichen Dosierungen, können sie zweckmässig beispielsweise als Herbizide zur Unkrautbekämpfung eingesetzt werden. Insbesondere zeigen die Herbizide gemäss der vorliegenden Erfindung eine ausgezeichnete selektive Bekämpfungswirkung bei Verwendung als Bodenbehandlungsmittel zur Vorauflaufbehandlung oder als Behandlungsmittel für Stengel, Blätter und Boden gegen Reisfeld-Unkräuter.

Wie oben angegeben wurde, zeichnen sich die Verbindungen der allgemeinen Formel (I) durch hohe Sicherheit aus und zeigen hervorragende herbizide Wirksamkeit.

Weiterhin besitzen die Verbindungen der allgemeinen Formel (I) neben der ausgezeichneten Sicherheit und der hervorragenden herbiziden Aktivität ein breites herbizides Spektrum.

Beispielsweise entfalten diese Verbindungen eine signifikante herbizide Wirkung gegenüber den nachstehenden Reisfeld-Unkräutern ohne irgendwelche schädlichen Effekte gegenüber Reispflanzen:

| *Pflanzenname:* | *Lateinische wissenschaftliche Bezeichnung:* |
|---|---|
| *Dikotyledonen* | |
| Kikashigusa | Rotala indica Koehne |
| Falsche Pimpernelle (Büchsenkraut) | Lindernia procumbens Philcox |
| Falscher Weiderich (Heusenkraut) | Ludwigia prostrata Roxburgh |
| Breitblättriges Laichkraut | Potamogeton distinctus A. Bennett |
| Amerikanische Wasserwurz (Kreuztännel) | Elatine triandra Schk. |
| *Monokotyledonen* | |
| Hühnerhirse | Echinochloa crus-galli Beau. |
| Monochoria | Monochoria vaginalis Presl. |
| Nadel-Sumpfbinse | Eleocharis acicularis L. |
| Wassernuss | Eleocharis kuroguwai Ohwi |
| Schirmkraut (Cypergras) | Cyperus difformis L. |
| Mizugayatsuri (Cypergras) | Cyperus serotinus Rottboell |
| Urikawa (Pfeilkraut) | Sagittaria pygmaea Miq. |
| Schmallblättriger Wasserwegerich (Froschlöffel) | Alisma canaliculatum A. Braun et Bouché |
| Simse | Scirpus juncoides Roxburgh var. |

Weiterhin zeigen diese Verbindungen der Formel (I) eine signifikante herbizide Aktivität gegen die folgenden, auf höher gelegenen Anbauflächen wachsenden Unkräuter.

| *Pflanzenname:* | *Lateinische wissenschaftliche Bezeichnung:* |
|---|---|
| *Dikotyledonen* | |
| Knöterich | Polygonum sp. |
| Gänsefuss | Chenopodium album Linnaeus |
| Vogelmiere | Stellaria media Villars |
| Kohl-Portulak | Portulaca oleracea Linnaeus |
| *Monokotyledonen* | |
| Hühnerhirse | Echinochloa crus-galli Beauv. var. |
| Fingergras | Digitaria adscendens Henr. |
| Riedgras | Cyperus iria L. |

Die im Vorstehenden angegebenen Pflanzennamen sind typische Beispiele für die jeweils mit der lateinischen wissenschaftlichen Bezeichnung genannte Gattung.

Die Einsetzbarkeit der durch die allgemeine Formel (I) bezeichneten Verbindungrn gemäss der vorliegenden Erfindung ist nicht auf Unkräuter in bewässerten Reisfeldern und auf höher gelegenen landwirtschaftlichen Anbauflächen beschränkt, sondern sie sind auch wirksam gegen Unkräuter, die die Weichbinsen (Mattenbinsen) schädigen, Unkräuter, die auf zeitweise brach liegenden Ländereien auftreten usw. Die hierin verwendete Bezeichnung «Unkräuter» bezeichnet im weitesten Sinne alle Pflanzen, die an Stellen auftreten, wo sie unerwünscht sind.

Die Verbindungen der allgemeinen Formel (I) gemäss der vorliegenden Erfindung besitzen auch starke fungizide Aktivität gegen einen breiten Bereich von Fungi, die Pflanzenerkrankungen verursachen, und auch eine ausgezeichnete Restwirkung und können aus diesem Grunde zur Bekämpfung von Pflanzenkrankheiten eingesetzt werden. Das fungizide Spektrum dieser Verbindungen zeigt, dass sie in wirksamer Weise zur Bekämpfung von Pflanzenkrankheiten verwendet werden können, die beispielsweise durch Archimycetes, Phycomycetes, Ascomycetes, Basidiomycetes und Fungi imperfecti sowie durch verschiedenartige Bakterien verursacht werden.

Von speziellem Wert sind die Verbindungen gemäss der vorliegenden Erfindung beispielsweise zur Bekämpfung der Brusone-Krankheit von Reis (Piricularia oryzae), Reis-Braunfleckenkrankheit (Ophiobolus miyabeanus), des Schwarzpilz des Kohls (Alternaria brassicae), der Alternaria-Blattfleckenkrankheit des Apfels (Alternaria mali), der Alternaria-Blattfleckenkrankheit der Birne (Alternaria kikuchiana), der Phytophthora-Braunfäule verschiedener Nutzpflanzen (Phytophthora infestans) und der Brennfleckenkrankheit der Gurke bzw. der östlichen Melonen zum Einlegen (Colletotrichum lagenarium). Auch Reis-Bakteriose (Xanthomonas oryzae) ist hier zu nennen, die eine bedeutsame Bakterienkrankheit ist.

Zur Anwendung als Herbizide oder Fungizide für Landwirtschaft und Gartenbau können die Verbindungen gemäss der vorliegenden Erfindung in Form verschiedener Formulierungen eingesetzt werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Einsatz werden diese verschiedenen Formulierungen entweder unmittelbar oder nach dem Verdünnen mit Wasser auf die gewünschte Konzentration zur Anwendung gebracht.

Beispiele für die landwirtschaftlich unbedenklichen Hilfsstoffe, auf die hier Bezug genommen wird, umfassen Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe [z.B. n-Hexan, Petrolether, Erdöl-Fraktionen (z.B. Paraffinwachse, Kerosin, Leichtöle, Mittelöle und Schweröle), Benzol, Taluol und Xylol], halogenierte Kohlenwasserstoffe (z.B. Methylenchlorid, Kohlenstofftetrachlorid, Ethylenchlorid, Ethylendibromid, Chlorbenzol und Chloroform), Alkohole (z.B. Methanol, Ethanol, Propanol und Ethylenglycol), Ether (z.B. Diethylether, Ethylenoxid und Dioxan), Alkohol-ether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidemehle, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für oberflächenaktive Mittel umfassen anionische oberflächenaktive Mittel wie Alkylsulfonsäureester (z.B. Natriumlaurylsulfat), Arylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchlorid) und Polyoxyethylenalkylamine, nicht-ionische oberflächenaktive Mittel wie polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acyl-Haftmittel), Aerosol-Treibmittel (z.B. Trichlorofluoromethan, Dichlorofluoromethan, 1,2,2-Trichloro-1,1,2-trifluoroethan, Chlorbenzol, verflüssigtes Erdgas und niedere Ether), die Verbrennung steuernde Mittel für Räucherpräparate (z.B. Nitrite, Zink-Pulver und Dicyndiamid), Sauerstoff abgebende Mittel (z.B. Chlorate), wirkungsverlängernde Mittel, Dispersions-Stabilisatoren [z.B. Casein, Tragant, Carboxymethylcellulose (CMC) und Polyvinylalkohol (PVA)] und synergistische Mittel.

Die Verbindungen gemäss der vorliegenden Erfindung können mittels allgemein bei der Herstellung von Agrochemikalien angewandter Methoden zu verschiedenartigen Präparaten formuliert werden. Erläuternde Beispiele für solche Anwendungsformen sind emulgierbare Konzentrate, Öl-Präparate, benetzbare Pulver, lösliche Pulver, Suspensionen, Stäubemittel, Granulat, Pulverpräparate, Räuchermittel, Tabletten, Aerosole, Pasten und Kapseln.

Die Herbizide oder land- und gartenwirtschaftlichen Fungizide gemäss der vorliegenden Erfindung können etwa 0,001 bis etwa 100 Gew.-%, vorzugsweise etwa 0,005 bis etwa 95 Gew.-% des vorerwähnten aktiven Wirkstoffes enthalten.

Für den praktischen Gebrauch beträgt die geeignete Menge der aktiven Verbindung in den vorgenannten Mitteln in ihren verschiedener Formen und gebrauchsfertigen Präparaten beispielsweise etwa 0,01 bis etwa 95 Gew.-%, vorzugsweise etwa 0,05 bis etwa 60 Gew.-%.

Der Gehalt des Wirkstoffs kann in geeigneter Weise je nach der Form des Präparats oder Mittels, dem Verfahren, Zweck, der Zeit und dem Ort seiner Anwendung, dem Zustand des Auftretens von Unkräutern oder Pflanzenkrankheiten usw. variiert werden.

Erforderlichenfalls können die Verbindungen gemäss der vorliegenden Erfindung weiterhin auch in Kombination mit anderen Agrochemikalien verwendet werden, beispielsweise mit Insektiziden, anderen Fungiziden, Mitiziden, Nematiziden, Anti-Virus-Mitteln, anderen Herbiziden, Pflanzen-Wachstumsregulatoren und Lockstoffen [z.B. Organophosphat-Verbindungen, Carbamat-Verbindungen, Dithio(oder thiol)carbamat-Verbindungen, organischen Chlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen] und/oder Düngemitteln.

Die den im Vorstehenden bezeichneten Wirkstoff gemäss der Erfindung enthaltenden veschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Giessen usw.), Räuchern, Bodenbehandlung (Vermischen mit dem Boden, Streuen, Bedampfen, Giessen usw.), Oberflächenbehandlung (Beschichten, Bändern, Bestäuben, Bedecken usw.) und Eintauchen. Sie können auch mittels des sogenannten Ultra-Low-Volume-Sprühverfahrens aufgebracht werden.

Nach diesem Verfahren kann der Wirkstoff sogar in einer Konzentration von 100% zur Anwendung gelangen.

Die Aufwandmengen pro Flächeneinheit betragen beispielsweise etwa 0,1 bis etwa 8 kg/ha, vorzugsweise etwa 0,25 bis etwa 5 kg/ha für das Herbizid und etwa 1 bis etwa 10 kg/ha für das land- und gartenwirtschaftliche Fungizid. In besonders gelagerten Fällen können oder sollten sogar die Aufwandmengen ausserhalb des angegebenen Bereich liegen.

Gemäss der vorliegenden Erfindung kann ein herbizides Mittel oder ein fungizides Mittel für Landwirtschaft und Gartenbau verfügbar gemacht werden, das eine Verbindung der allgemeinen Formel (I) als Wirkstoff und ein Verdünnungsmittel (ein Lösungsmittel und/oder ein Streckmittel und/oder einen Träger) und/oder ein oberflächenaktives Mittel und, sofern weiter erforderlich, einen Stabilisator, ein Haftmittel, ein synergistisches Mittel usw. enthält.

Die vorliegende Erfindung stellt ebenfalls ein Verfahren zur Bekämpfung von Unkräutern und Erkrankungen von Nutzpflanzen zur Verfügung, bei dem auf Unkräuter, Pflanzenpathogene und/oder den Ort ihres Auftretens eine Verbindung der allgemeinen Formel (I) allein oder im Gemisch mit einem Verdünnungsmittel (einem Lösungsmittel und/oder einem Streckmittel und/oder einem Träger) und/oder einem oberflächenaktiven Mittel und, sofern weiter erforderlich, einem Stabilisator, einem Haftmittel, einem synergistischen Mittel usw. aufgebracht wird.

Die folgenden Beispiele erläutern die vorliegende Erfindung im einzelnen. Es sei jedoch darauf hingewiesen, dass die vorliegende Erfindung nicht allein auf diese speziellen Beispiele beschränkt ist.

*Herstellungsbeispiele*

*Beispiel 1*

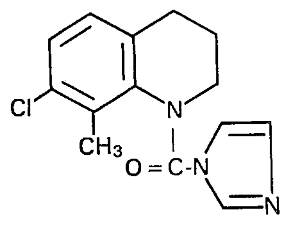

(Verbindung Nr. 1)

7-Chloro-8-methyl-1,2,3,4-tetrahydrochinolin (1,8 g) und N,N'-Carbonyldiimidazol (1,6 g) wurden in Toluol (20 ml) 24 h unter Rühren erhitzt. Das Lösungsmittel wurde unter vermindertem Druck abgedampft, und der Rückstand wurde bei 80°C und 1,33 mbar (1 mmHg) gehalten. Wasser wurde dem Rückstand zugesetzt, um diesen zur Kristallisation zu bringen. Die Kristalle wurden durch Filtration gesammelt, wodurch das gewünschte 7-Chloro-8-methyl-1(H),2,3,4-tetrahydrochinolin-1-yl-carbonylimidazol (2,1 g) erhalten wurde. Das Produkt kristallisiert aus Ether; Schmp. 115-117°C.

*Beispiel 2*

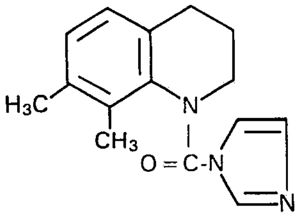

(Verbindung Nr. 29)

7,8-Dimethyl-1(H),2,3,4-tetrahydrochinolin-1-yl--carbonylchlorid (2,2 g) und Imidazol (2,0 g) wurden in Tetrahydrofuran (20 ml) 16 h unter Rückflusstemperatur erhitzt. Das Tetrahydrofuran und das überschüssige Imidazol wurden unter vermindertem Druck abgedampft. Wasser wurde dem Rückstand zugesetzt, um diesen zur Kristallisation zu bringen. Die Kristalle wurden durch Filtration gesammelt, wodurch das gewünschte 7,8-Dimethyl-1(H),2,3,4-tetrahydrochinolin-1-ylcarbonylimidazol (2,2 g) erhalten wurde. Das Produkt wird aus Ether umkristallisiert; Schmp. 122-124°C.

Die folgende Tabelle 1 zeigt die Verbindungen der allgemeinen Formel (I) gemäss der vorliegenden Erfindung, die nach den gleichen Verfahrensweisen, wie sie in den Beispielen 1 und 2 beschrieben sind, hergestellt wurden.

TABELLE 1

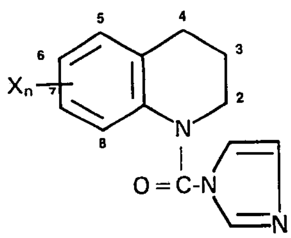

| Verbindung Nr. | Xn (Physikal. Konstanten) |
|---|---|
| 2 | 6-$CH_3$ (Schmp. 78-81°) |
| 3 | 6-$C_2H_5$ |
| 4 | 6-$C_3H_7$-iso |
| 5 | 6-$C_4H_9$-tert |
| 6 | 7-$CH_3$ (Schmp. 125-127°C) |
| 7 | 8-$CH_3$ (Schmp. 107-108°C) |
| 8 | 8-$C_2H_5$ (Schmp. 82-84°C) |
| 9 | 8-$C_3H_7$-n |
| 10 | 8-F (Schmp. 95-98°C) |
| 11 | 8-Br (Schmp. 136-140°C) |
| 12 | 5-F |
| 13 | 6-F |
| 14 | 7-F |
| 15 | 6-Br |
| 16 | 7-Br |
| 17 | 8-I |
| 18 | 6-$NO_2$ (Schmp. 127-130°C) |
| 19 | 8-$NO_2$ |
| 20 | 8-CN |

TABELLE 1 - Fortsetzung

| Verbindung Nr. | Xn (Physikal. Konstanten) |
|---|---|
| 21 | 8-$CF_3$ (Schmp. 97-98°C) |
| 22 | 8-$OCH_3$ (Schmp. 126-129°C) |
| 23 | 6-$OCH_3$ |
| 24 | 6-$C_3H_7$-iso |
| 25 | 7-$OCH_3$ |
| 26 | 7-$OC_3H_7$-iso |
| 27 | 8-$OC_2H_5$ (Schmp. 101-103°C |
| 28 | 6,8-$(CH_3)_2$ (Schmp. 121-123°C) |
| 30 | 6,7-$(CH_3)_2$ (Schmp. 112-114°C) |
| 31 | 6,7,8-$(CH_3)_3$ |
| 32 | 6-Cl, 8-$CH_3$ (Schmp. 138-141°C) |
| 33 | 6-$CH_3$, 8-Cl (Schmp. 140-143°C) |
| 34 | 6-Cl, 8-$CF_3$ (Schmp. 112-115°C) |
| 35 | 5-$CH_3$, 8-Cl |
| 36 | 6-$CH_3$, 7-Cl |
| 37 | 7-$CH_3$, 8-Cl (Schmp. 85-87°C) |
| 38 | 5-Cl, 8-$CH_3$ |
| 39 | 6-Br, 8-$CH_3$ |
| 40 | 5-Cl, 8-$OCH_3$ |
| 41 | 6,8-$Cl_2$, 5-$C_3H_7$-iso |
| 42 | 6-Cl, 8-F, 5-$C_3H_7$-iso |
| 43 | 6,8-$F_2$ (Schmp. 87-88°C) |
| 44 | 7,8-$F_2$ (Schmp. 78-81°C) |
| 45 | 6-$CF_3$ |
| 46 | 7-$CF_3$ |
| 47 | 5-Cl, 8-$CF_3$ |
| 48 | 6-Cl, 7-$CF_3$ |
| 49 | 7-Cl, 8-$CF_3$ |
| 50 | 6,7-$F_2$ |
| 51 | 6,8-$Br_2$ |
| 52 | 7,8-$Br_2$ |
| 53 | 6-F, 8-Cl |
| 54 | 6-Cl, 8-F |
| 55 | 7-F, 8-Cl |
| 56 | 6-Br, 8-Cl |
| 57 | 6,7-$(OCH_3)_2$ |
| 58 | 7,8-$(OCH_3)_2$ |
| 59 | 6-$SCH_3$ |
| 60 | 8-$SCH_3$ |
| 61 | 8-$SC_2H_5$ |
| 62 | 6-$SCF_3$ |
| 63 | 8-$SCF_3$ |
| 64 | 8-$SCF_2Cl$ |
| 65 | 6-$OCF_3$ |
| 66 | 8-$OCF_3$ |
| 67 | 8-$OCHF_2$ |
| 68 | 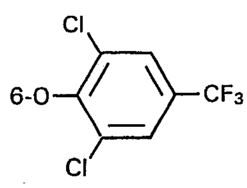 |
| 69 | 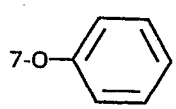 |
| 70 | 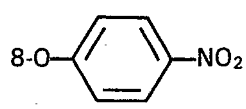 |

TABELLE 1 - Fortsetzung

| Verbindung Nr. | Xn (Physikal. Konstanten) |
|---|---|
| 71 | 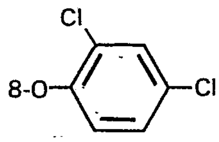 |
| 72 | 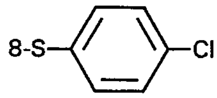 |
| 73 | 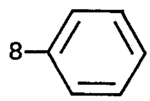 |
| 74 | 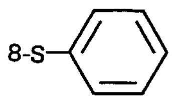 |
| 75 | 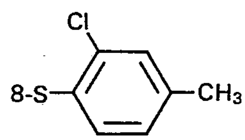 |
| 76 | Formel: 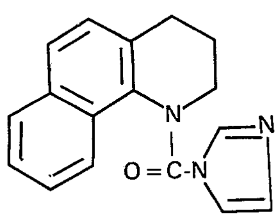 (Schmp. 156-159°C) |
| 77 | $8-C_3H_7$-iso (Schmp. 98-100°C) |
| 78 | $8-C_4H_9$-tert |
| 79 | $8-OC_3H_7$-iso |
| 80 | 7-F, $8-CH_3$ (Schmp. 114-115°C) |
| 81 | O<br>‖<br>$8-S-CH_3$<br>‖<br>O |
| 82 | 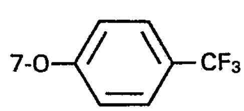 |

TABELLE 1 - Fortsetzung

| Verbindung Nr. | Xn (Physikal. Konstanten) |
|---|---|
| 83 | Formel: 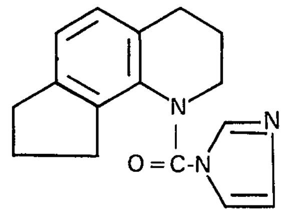 |
| 84 | Formel: 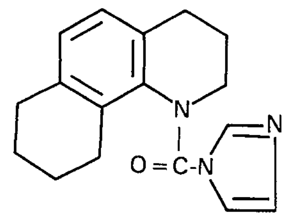 (Schmp. 125-127°C) |

*Beispiel 3*

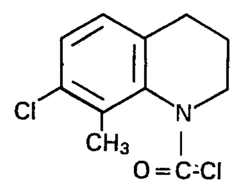

(Verbindung Nr. III-1)

Eine Lösung von Chlorameisensäuretrichloromethylester (20 ml) in Ethylacetat (100 ml) wurde auf 50°C bis 60°C (Ölbad-Temperatur) erwärmt, und unter Rühren wurde eine Lösung von 7-Chloro-8--methyl-1,2,3,4-tetrahydrochinolin (9 g) in Ethylacetat (20 ml) im Laufe von 30 min tropfenweise hinzugefügt. Die Reaktionstemperatur wurde allmählich auf Rückflusstemperatur gesteigert, und die Mischung wurde 5 h gerührt. Das Lösungsmittel wurde auf einem Dampfbad abgedampft, und danach wurde der Rückstand unter Hochvakuum destilliert, wonach das gewünschte 7-Chloro-8-methyl-1,2,3,4--tetrahydrochinolin-1-yl-carbonylchlorid (9,5 g) erhalten wurde; Sdp. 165-170°C/0,53 mbar (0,4 mmHg).

Die Verbindungen der allgemeinen Formel (III) gemäss der vorliegenden Erfindung, die nach der gleichen Verfahrensweise, wie sie in Beispiel 3 beschrieben ist, hergestellt wurden, sind in der nachstehenden Tabelle 2 aufgeführt.

TABELLE 2

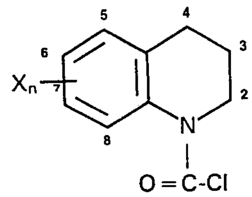

| Verbindung Nr. | Xn (Physikal. Konstanten) |
|---|---|
| III - 2 | 6-$CH_3$ [(Sdp. 108-111°C/ 0,13 mbar (0,1 mmHg)] |
| III - 3 | 6-$C_2H_5$ |
| III - 4 | 6-$C_3H_7$-iso |
| III - 5 | 6-$C_4H_9$-tert |
| III - 6 | 7-$CH_3$ [Sdp. 105-110°C/ 13 mbar (0,1 mmHg)] |
| III - 7 | 8-$CH_3$ [Sdp. 114-116°C/ 0,27 mbar (0,2 mmHg)] |
| III - 8 | 8-$C_2H_5$ [Sdp. 100-103°C/ 0,11 mbar (0,08 mmHg)] |
| III - 9 | 8-$C_3H_7$-n |
| III - 10 | 8-F [Sdp. 120-122°C/ 0,33 mbar (0,25 mmHg)] |
| III - 11 | 8-Br [Sdp. 140-144°C/ 0,4 mbar (0,3 mmHg)] |
| III - 12 | 5-F |
| III - 13 | 6-F |
| III - 14 | 7-F |
| III - 15 | 6-Br |
| III - 16 | 7-Br |
| III - 17 | 8-I |
| III - 18 | 6-$NO_2$ Öl |
| III - 19 | 8-$NO_2$ |
| III - 20 | 8-CN |
| III - 21 | 8-$CF_3$ (Schmp. 48-51°C) |
| III - 22 | 8-$OCH_3$ [Sdp. 143-145°C/ 0,4 mbar (0,3 mmHg)] |
| III - 23 | 6-$OCH_3$ |
| III - 24 | 6-$C_3$-$H_7$-iso |
| III - 25 | 7-$OCH_3$ |
| III - 26 | 7-$OC_3H_7$-iso |
| III - 27 | 8-$OC_2H_5$ [Sdp. 168-171°C/ 0,67 mbar (0,5 mmHg)] |
| III - 28 | 6,8-$(CH_3)_2$ [Sdp. 129-131°C/ 0,20 mbar (0,15 mmHg)] |
| III - 29 | 7,8-$(CH_3)_2$ [Sdp. 125-130°C/ 0,20 mbar (0,15 mmHg)] |
| III - 30 | 6,7-$(CH_3)_2$ [Sdp. 133-137°C/ 0,4 mbar (0,3 mmHg)] |
| III - 31 | 6,7,8-$(CH_3)_3$ |
| III - 32 | 6-Cl, 8-$CH_3$ [Sdp. 173-175°C/ 0,67 mbar (0,5 mmHg)] |
| III - 33 | 6-$CH_3$, 8-Cl [Sdp. 188-189°C/ 0,6 mbar (0,6 mmHg)] |
| III - 34 | 6-Cl, 8-$CF_3$ [Sdp. 138-139°C/ 0,27 mbar (0,2 mmHg)] |
| III - 35 | 5-$CH_3$, 8-Cl |
| III - 36 | 6-$CH_3$, 7-Cl |

TABELLE 2 - Fortsetzung

| Verbindung Nr. | Xn (Physikal. Konstanten) |
|---|---|
| III - 37 | 7-$CH_3$, 8-Cl [Sdp. 160°C/ 0,53 mbar (0,4 mmHg)] |
| III - 38 | 5-Cl, 8-$CH_3$ |
| III - 39 | 6-Br, 8-$CH_3$ |
| III - 40 | 5-Cl, 8-$OCH_3$ |
| III - 41 | 6,8-$Cl_2$, 5-$C_3H_7$-iso |
| III - 42 | 6-Cl, 8-F, 5-$C_3H_7$-iso |
| III - 43 | 6,8-$F_2$ [Sdp.110-115°C/ 1,07 mbar (0,08 mmHg)] |
| III - 44 | 7,8-$F_2$ [Sdp. 108-110°C/ 1,07 mbar (0,08 mmHg)] |
| III - 45 | 6-$CF_3$ |
| III - 46 | 7-$CF_3$ |
| III - 47 | 5-Cl, 8-$CF_3$ |
| III - 48 | 6-Cl, 7-$CF_3$ |
| III - 49 | 7-Cl, 8-$CF_3$ |
| III - 50 | 6,7-$F_2$ |
| III - 51 | 6,8-$Br_2$ |
| III - 52 | 7,8-$Br_2$ |
| III - 53 | 6-F, 8-Cl |
| III - 54 | 6-Cl, 8-F |
| III - 55 | 7-F, 8-Cl |
| III - 56 | 6-Br, 8-Cl |
| III - 57 | 6,7-$(OCH_3)_2$ |
| III - 58 | 7,8-$(OCH_3)_2$ |
| III - 59 | 6-$SCH_3$ |
| III - 60 | 8-$SCH_3$ |
| III - 61 | 8-$SC_2H_5$ |
| III - 62 | 6-$SCF_3$ |
| III - 63 | 8-$SCF_3$ |
| III - 64 | 8-$SCF_2Cl$ |
| III - 65 | 6-$OCF_3$ |
| III - 66 | 8-$OCF_3$ |
| III - 67 | 8-$OCHF_2$ |
| III - 68 | 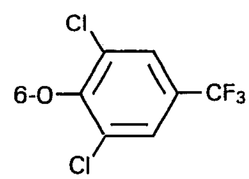 |
| III - 69 | 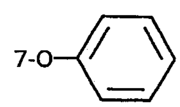 |
| III - 70 | 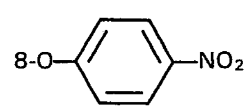 |
| III - 71 | 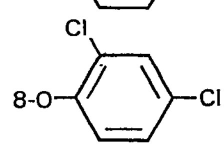 |
| III - 72 | 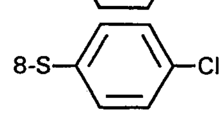 |
| III - 73 | 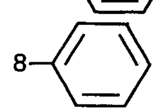 |

TABELLE 2 - Fortsetzung

| Verbindung Nr. | Xn (Physikal. Konstanten) |
|---|---|
| III - 74 | 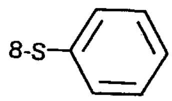 |
| III - 75 | 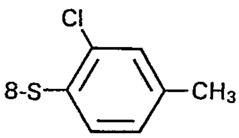 |
| III - 76 | Formel: 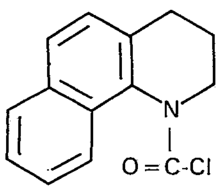 [Sdp. 155-157°C/ 0,67 mbar (0,5 mmHg)] |
| III - 77 | 8-$C_3H_7$-iso [Sdp. 152-154°C/ 0,67 mbar (0,5 mmHg)] |
| III - 78 | 8-$C_4H_9$-tert |
| III - 79 | 8-$OC_3H_7$-iso |
| III - 80 | 7-F, 8-$CH_3$ [Sdp. 151-152°C/ 0,67 mbar (0,5 mmHg)] |
| III - 81 | 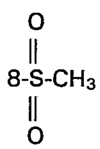 [Sdp. 200°C/1,33 mbar (1 mmHg)] |
| III - 82 | 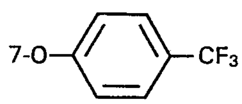 |
| III - 83 | Formel: 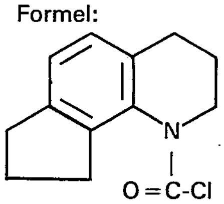 |
| III - 84 | Formel: 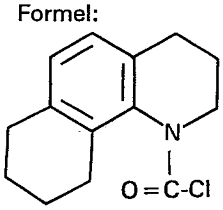 [Sdp. 195-198°C/ 0,67 mbar (0,5 mmHg)] |

*Beispiel 4*

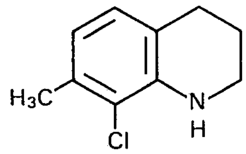

Platinoxid (0,5 g) wurde zu einer Lösung (200 ml) von 8-Chloro-7-methylchinolin (17,75 g) in Methanol hinzugefügt. Die Mischung wurde in einen Autoklaven (Innenvolumen: 500 ml) überführt, und Wasserstoff-Gas wurde eingeleitet, bis der Innendruck 19,62 bar (20 kg/cm$^2$) betrug. Die Mischung wurde 24 h bei Raumtemperatur gerührt und danach filtriert. Das Lösungsmittel (Methanol) wurde unter vermindertem Druck abgedampft, wonach 8-Chloro-7-methyl-1,2,3,4-tetrahydrochinolin (18 g) erhalten wurde. $n_D^{20}$ 1,5912.

In der gleichen Weise, wie in Beispiel 4 angegeben ist, wurde 7-Chloro-8-methyl-1,2,3,4-tetrahydrochinolin (Schmp. 53-55°C) synthetisiert.

*Verwendungsbeispiele*

Die bekannte Vergleichs-Verbindung wird wie folgt identifiziert:

*Vergleichs-Verbindung A-1*

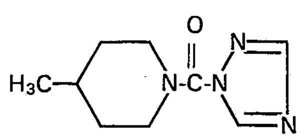

(bekannt aus der US-PS 3 308 131).

*Beispiel 5*

*Prüfung der Wirkung gegen im Wasser wachsende Reisfeld-Unkräuter durch Behandlung des Bodens sowie der Stengel und Blätter unter Bewässerungs-Bedingungen (Topf-Test):*

Herstellung des Wirkstoff-Präparats:

Träger: 5 Gewichtsteile Aceton;
Emulgator: 1 Gewichtsteil Benzyloxypolyglykolether.

Zur Herstellung eines Wirkstoff-Präparats wird 1 Gew.-Teil jeder der aktiven Verbindungen mit dem Träger und Emulgator in den vorbezeichneten Mengen vermischt, und eine vorher festgelegte Menge des resultierenden emulgierbaren Konzentrats wurde mit Wasser verdünnt.

Test-Verfahren:

Reis-Kulturboden wurde in Wagner-Töpfe (2 dm$^2$) gefüllt, und zwei Reis-Setzlinge (Varietät: Kinnampu) im 2- bis 3-Blattstadium (Pflanzenhöhe etwa 10 cm) wurden in jeden Topf umgepflanzt. Samen von Echinochloa crus-galli, Cyperus microiria, Monochoria vaginalis, Scirpus juncoides und breitblättrigen

Unkräutern, kleine Stücke von Eleocharis acicularis und Knollen von Cyperus serotinus wurden in die Töpfe eingesät bzw. eingesetzt. Der Boden in den Töpfen wurde in feuchtem Zustand gehalten. Nachdem Echinochloa crus-galli bis etwa zum 2-Blatt-Stadium gewachsen war (etwa 7 bis 9 Tage nach der Einsaat) wurden die Töpfe mit Wasser bis zu einer Tiefe von etwa 6 cm gefüllt. Eine vorher festgelegte Menge des Wirkstoffs wurde in Form einer Emulsion mittels einer Pipette dem Wasser zugegeben. Nach der Behandlung wurden die Töpfe im Laufe von zwei Tagen mit einer Geschwindigkeit von 2 bis 3 cm pro Tag entwässert, während das Wasser aus den Töpfen heraussickern konnte. Danach wurde die Wassertiefe in den Töpfen bei etwa 3 cm gehalten. In der vierten Woche nach der Behandlung mit der Chemikalie wurden die herbizide Wirkung und der Grad der Phytotoxizität mit Hilfe einer Skala von 0 bis 5 nach dem folgenden Massstab bewertet.

| Bewertung | der herbiziden Wirkung (Herbizid-Rate, bezogen auf die unbehandelte Fläche) | |
|---|---|---|
| 5 | wenigstens 95% | (verdorrt) |
| 4 | wenigstens 80%, | jedoch weniger als 95% |
| 3 | wenigstens 50%, | jedoch weniger als 80% |
| 2 | wenigstens 30%, | jedoch weniger als 50% |
| 1 | wenigstens 10%, | jedoch weniger als 30% |
| 0 | weniger als 10% | (unwirksam) |

| Bewertung | der Phytotoxizität gegenüber im Wasser befindlichem Reis (Phytotoxizitätsrate, bezogen auf die unbehandelte Fläche) | |
|---|---|---|
| 5 | wenigstens 90% | (Ausrottung) |
| 4 | wenigstens 50%, | jedoch weniger als 90% |
| 3 | wenigstens 30%, | jedoch weniger als 50% |
| 2 | wenigstens 10%, | jedoch weniger als 30% |
| 1 | mehr als 0%, | jedoch weniger als 10% |
| 0 | 0% | (keine Phytotoxizität) |

Die Unkräuter sind durch die nachstehenden Abkürzungen A, B, C, D, E, F und G bezeichnet.

A: Echinochloa crus-galli

B: Eleocharis acicularis

C: Cyperus microiria

D: Scirpus juncoides

E: Monochoria vaginalis

F: Breitblättrige Unkräuter (darunter Lindernia procumbens, Rotala indica, Elatine triandra usw.)

G: Cyperus serotinus

TABELLE 3

| Verbindung Nr. | Wirkstoff-Menge kg/ha | Herbizide Wirkung Unkräuter A | B | C | D | E | F | G | Phytotoxizität gegen Reis |
|---|---|---|---|---|---|---|---|---|---|
| 1 | 1,0 | 5 | 5 | 5 | 5 | 5 | 5 | 5 | 0 |
| 6 | 1,0 | 5 | 5 | 5 | 5 | 5 | 5 | 5 | 0 |
| 8 | 1,0 | 5 | 5 | 5 | 5 | 5 | 5 | 5 | 0 |
| 11 | 1,0 | 4 | 5 | 5 | 5 | 5 | 5 | 5 | 0 |
| 14 | 1,0 | 5 | 5 | 5 | 5 | 5 | 5 | 5 | 0 |
| 16 | 1,0 | 5 | 5 | 5 | 5 | 5 | 5 | 5 | 0 |
| 21 | 1,0 | 4 | 5 | 5 | 5 | 5 | 5 | 5 | 0 |
| 22 | 1,0 | 4 | 5 | 5 | 5 | 5 | 5 | 5 | 0 |
| 25 | 1,0 | 4 | 5 | 5 | 5 | 5 | 5 | 5 | 0 |
| 29 | 1,0 | 5 | 5 | 5 | 5 | 5 | 5 | 5 | 0 |
| 32 | 1,0 | 4 | 5 | 5 | 5 | 5 | 5 | 5 | 0 |
| 33 | 1,0 | 4 | 5 | 5 | 5 | 5 | 5 | 4 | 0 |
| 37 | 1,0 | 5 | 5 | 5 | 5 | 5 | 5 | 5 | 0 |
| 39 | 1,0 | 4 | 5 | 5 | 5 | 5 | 5 | 4 | 0 |
| 44 | 1,0 | 5 | 5 | 5 | 5 | 5 | 5 | 5 | 0 |
| 52 | 1,0 | 5 | 5 | 5 | 5 | 5 | 5 | 5 | 0 |
| 58 | 1,0 | 4 | 5 | 5 | 5 | 5 | 5 | 4 | 0 |
| 60 | 1,0 | 4 | 5 | 5 | 5 | 5 | 5 | 4 | 0 |
| 66 | 1,0 | 4 | 5 | 5 | 5 | 5 | 5 | 4 | 0 |
| Vergleich: A-1 | 2,0 | 0 | 0 | 1 | 0 | 0 | 1 | 0 | 0 |

*Beispiel 6*

*Prüfung der Wirkung gegen Cochliobolus miyabeanus, Sprühen auf Stengel und Blätter*

Reispflanzen (Varietät Kusabue) wurden in unglasierten Töpfen mit einem Durchmesser von 12 cm gezogen, und im Drei- bis Vierblatt-Stadium wurde eine gemäss dem folgenden Beispiel 10 hergestellte Verdünnung der Test-Verbindung in einer Dosis von 50 ml auf jeweils drei Töpfe aufgesprüht. Am folgenden Tag wurde eine Suspension künstlich kultivierter Sporen von Cochliobolus miyabeanus zweimal auf die Töpfe aufgesprüht. Die Töpfe wurden zur Auslösung der Infektion in einem Raum mit einer relativen Luftfeuchtigkeit von 100% und einer Temperatur von 25°C stehen gelassen. Sieben Tage nach der Inokulierung wurde der Grad der Erkrankungen pro Topf mittels einer Skala von 0 bis 5 wie folgt bewertet, und der Bekämpfungsindex wurde berechnet.

| *Grad der Erkrankung* | *Ausmass der Erkrankung* |
|---|---|
| 0 | keine Erkrankung |
| 1 | geringfügig |
| 2 | klein |
| 3 | mittel |
| 4 | gross |
| 5 | sehr gross |

$$\text{Bekämpfungs-Index (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

In diesem Test bildeten jeweils drei Töpfe eine Partie. Im Ergebnis zeigte beispielsweise die Verbindung Nr. 10 einen Bekämpfungs-Index von 100% bei einer Konzentration der aktiven Komponente von 500 ppm. Die Vergleichs-Verbindung A-1 zeigte bei 500 ppm einen Bekämpfungs-Index von weniger als 100%.

*Beispiel 7*

*Test auf Bekämpfung der Braunfäule der Tomate (Phytophthora infestans)*

Jede der Test-Verbindungen in Form einer Emulsion, die gemäss dem folgenden Beispiel 10 hergestellt worden war, wurde auf in unglasierten 9 cm-Töpfen gezogene Tomaten (Varietät: «Kurihara») mit einer Sprühpistole aufgesprüht. Einen Tag nach dem Sprühen wurden die Pflanzen mit einer Sporen-Suspension des angegebenen Pathogens inokuliert, und die Töpfe wurden über Nacht in einem Raum mit einer konstanten Temperatur von 22°C und einer Luftfeuchtigkeit von wenigstens 90% stehen gelassen. Fünf Tage später wurde der Grad der Erkrankung mittels der nachfolgend angegebenen Standards durch das Verhältnis der Läsionen aufweisenden Flächen bestimmt, und der Bekämpfungsindex wurde berechnet.

| *Grad der Erkrankung* | *Verhältnis der Fläche der Verletzungen (%)* |
|---|---|
| 0 | 0 |
| 0,5 | 2 oder weniger |
| 1 | 3 bis 5 |
| 2 | 6 bis 15 |
| 3 | 16 bis 30 |
| 4 | 31 bis 50 |
| 5 | 51 oder mehr |

$$\text{Bekämpfungs-Index (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

Im Ergebnis zeigten die Verbindungen Nr. 7, 10, 28 und 34 einen Bekämpfungs-Index von 100% bei einer Konzentration der aktiven Komponente von 500 ppm. Die Vergleichs-Verbindung A-1 zeigte einen Bekämpfungs-Index von 0%.

*Beispiel 8*

*Test der Wirksamkeit gegen die Brusone-Krankheit von Reis durch Anwendung auf Stengel und Laub:*

Herstellung des Wirkstoff-Präparats:

| | |
|---|---|
| Aktive Verbindung: | 50 Gew.-Teile; |
| Träger: | 45 Gew.-Teile einer 1:5-Mischung aus Diatomeenerde und Kaolin; |
| Emulgator: | 5 Gew.-Teile Polyoxyethylenalkylenphenylether. |

Zur Herstellung eines benetzbaren Pulvers werden die aktive Verbindung, der Träger und der Emulgator in den angegebenen Mengen pulverisiert und vermischt. Eine vorher festgelegte Menge des Pulvers wurde mit Wasser verdünnt, wodurch die Test-Chemikalie hergestellt wurde.

Test-Verfahren:

Reispflanzen (Varietät: Asahi) wurden in unglasierten Töpfen mit einem Durchmesser von 12 cm kultiviert. Im 3- bis 4-Blatt-Stadium wurde eine Verdünnung einer Test-Verbindung, die wie oben angegeben mit vorher festgelegter Konzentration hergestellt worden war, auf die Töpfe in einer Aufwandmenge von 50 ml für jeweils 3 Töpfe aufgesprüht. Am nächsten Tage wurde eine Suspension künstlich kultivierter Sporen von Piricularia oryzae zweimal auf die Reispflanzen gesprüht, um den Pilz zu inokulieren. Die Töpfe wurden in einer feuchten Kammer bei einer Temperatur von 25°C und einer relativen Luftfeuchtigkeit von 100% gehalten, um eine Infektion zu bewirken. Sieben Tage nach der Inokulierung wurde der Grad der Erkrankung untersucht und nach dem folgenden Standard bewertet, und der Bekämpfungs-Index (%) wurde berechnet. Die Phytotoxizität wurde ebenfalls untersucht.

| *Grad der Erkrankung* | *Verhältnis der Fläche der Verletzungen (%)* |
|---|---|
| 0 | 0 |
| 0,5 | 2 oder weniger |
| 1 | 3 bis 5 |
| 2 | 6 bis 10 |
| 3 | 11 bis 20 |
| 4 | 21 bis 40 |
| 5 | 41 oder mehr |

$$\text{Bekämpfungs-Index (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

Im Ergebnis zeigten beispielsweise die Verbindungen Nr. 10, 22 und 32 einen Bekämpfungs-Index von 100% bei einer Konzentration der aktiven Komponente von 500 ppm. Die Vergleichs-Verbindung A-1 zeigte bei 500 ppm einen Bekämpfungs-Index von weniger als 10%.

*Beispiel 9 (Benetzbares Pulver)*

15 Teile der Verbindung Nr. 1 der Erfindung, 80 Teile eines 1:5-Gemisches aus pulvriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf Unkräuter, Pathogene und/oder den Ort ihres Auftretens aufgesprüht.

*Beispiel 10 (Emulgierbares Konzentrat)*

30 Teile der Verbindung Nr. 33 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf Unkräuter, Pathogene und/oder den Ort ihres Auftretens aufgesprüht.

*Beispiel 11 (Stäubemittel)*

2 Teile der Verbindung Nr. 3 der Erfindung und 98 Teile Tonpulver werden pulverisiert und gemischt, wodurch ein Stäubemittel hergestellt wird. Dieses wird über Unkräutern, Pathogenen und/oder dem Ort ihres Auftretens ausgestreut.

*Beispiel 12 (Stäubemittel)*

Die Verbindung Nr. 2 der Erfindung (1,5 Teile), 0,5 Teile Isopropyl-hydrogenphosphat (PAP) und 98 Teile Tonpulver werden pulverisiert und gemischt, wodurch ein Stäubemittel hergestellt wird. Dieses wird über Unkrätern, Pathogenen und/oder dem Ort ihres Auftretens ausgestreut.

*Beispiel 13 (Granulat)*

Wasser (25 Teile) wird zu einer Mischung aus 10 Teilen der Verbindung Nr. 1 der Erfindung, 30 Teilen Bentonit (Montmorillonit), 58 Teilen Talkum und 2 Teilen Lignosulfonat zugesetzt, und das Gemisch wird gut geknetet. Die Mischung wird mittels eines Extruder-Granulators zu einem Granulat mit einer Korngrösse von 0,43 bis 2,0 mm (10 bis 40 mesh) verarbeitet, das dann bei 40°C bis 50°C getrocknet wird, wodurch ein Granulat gebildet wird. Das Granulat wird über Unkräutern, Pathogenen und/oder dem Ort ihres Auftretens ausgestreut.

*Beispiel 14 (Granulat)*

Ein Drehmischer wird mit 95 Teilen Tonmineral-Teilchen mit einer Teilchengrössen-Verteilung zwischen 0,2 und 2 mm beschickt, und unter Drehen des Mischers werden 5 Teile der Verbindung Nr. 29 der Erfindung gelöst in einem organischen Lösungsmittel auf die Teilchen zur gleichmässigen Benetzung derselben aufgesprüht, wodurch ein Granulat gebildet wird. Das Granulat wird über Unkräutern, Pathogenen und/oder dem Ort ihres Auftretens ausgestreut.

**Patentanprüche**

1. Tetrahydrochinolin-1-ylcarbonylimidazol-Derivate der allgemeinen Formel (I)

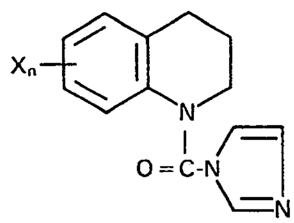

I

in der

X ein Halogen-Atom, eine $C_1$- bis $C_4$-Alkyl-Gruppe, eine Nitro-Gruppe, eine Cyano-Gruppe, eine Trifluoromethyl-Gruppe, eine $C_1$- bis $C_4$-Alkoxy-Gruppe, die durch ein Halogen-Atom substituiert sein kann, eine $C_1$- bis $C_4$-Alkylthio-Gruppe, die durch ein Halogen-Atom substituiert sein kann, eine $C_1$- bis $C_4$-Alkylsulfonyl-Gruppe, eine Phenyl-Gruppe, eine Phenylthio-Gruppe, die durch ein Halogen-Atom substituiert sein kann, oder eine Phenoxy-Gruppe, die durch wenigstens einen aus der aus einem Halogen-Atom, einer Trifluormethyl-Gruppe, einer Nitro-Gruppe und $C_1$- bis $C_4$-Alkyl-Gruppen bestehenden Klasse ausgewählten Substituenten substituiert sein kann, bezeichnet und

n 1, 2 oder 3 bezeichnet, und, sofern n 2 oder 3 ist, die zwei oder drei durch X bezeichneten Substituenten gleich oder verschieden sein können und zwei Substituenten X zusammen mit den Kohlenstoff-Atomen in den Positionen 7 und 8 des Tetrahydrochinolin-Rings einen gesättigten oder ungesättigten Kohlenwasserstoff-Ring mit 5 bis 6 Kohlenstoff-Atomen bilden können,

mit der Massgabe, dass, wenn n 1 ist, X kein Chlor-Atom ist, und wenn n 2 oder 3 ist, alle X nicht gleichzeitig Chlor-Atome sind.

2. Verbindungen nach Anspruch 1, dadurch gekennzeichnet, dass

X Fluor, Chlor, Brom, Alkyl mit 1 bis 4 Kohlenstoff-Atomen, Trifluoromethyl oder Alkoxy mit 1 bis 4 Kohlenstoff-Atomen, das durch Fluor und/oder Chlor substituiert sein kann, bezeichnet und

n 1 oder 2 bezeichnet, und, wenn n 2 ist, die beiden X identisch oder unterschiedlich sein können und zwei Substituenten X zusammen mit den Kohlenstoff-Atomen in den Positionen 7 und 8 des Tetrahydrochinolin-Rings einen gesättigten oder ungesättigten Kohlenwasserstoff-Ring mit 5 bis 6 Kohlenstoff-Atomen bilden können,

mit der Massgabe, dass, wenn n 1 ist, X kein Chlor ist, und wenn n 2 ist, beide X nicht gleichzeitig Chlor sind.

3. Verbindung nach Anspruch 1, dadurch gekennzeichnet, dass

X Fluor, Chlor, Methyl, Ethyl, n-Propyl, Isopropyl, Trifluoromethyl oder Methoxy, Ethoxy, n-Propoxy oder Isopropoxy, die durch Fluor und/oder Chlor substituiert sein können, bezeichnet und

n 2 bezeichnet und die beiden X identisch oder unterschiedlichen sein können und die zwei Substituenten X zusammen mit den Kohlenstoff-Atomen in den Positionen 7 und 8 des Tetrahydrochinolin-Rings einen gesättigten oder ungesättigten Kohlenwasserstoff-Ring mit 5 bis 6 Kohlenstoff-Atomen bilden können.

mit der Massgabe, dass, wenn n 2 ist, beide X nicht gleichzeitig Chlor sind.

4. Verfahren zur Herstellung von Tetrahydrochinolin-1-ylcarbonylimidazol-Derivaten der allgemeinen Formel (I)

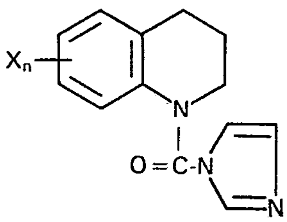

I

in der

X ein Halogen-Atom, eine $C_1$- bis $C_4$-Alkyl-Gruppe, eine Nitro-Gruppe, eine Cyano-Gruppe, eine Trifluoromethyl-Gruppe, eine $C_1$- bis $C_4$-Alkoxy-Gruppe, die durch ein Halogen-Atom substituiert sein kann, eine $C_1$- bis $C_4$-Alkylthio-Gruppe, die durch ein Halogen-Atom substituiert sein kann, eine $C_1$- bis $C_4$-Alkylsulfonyl-Gruppe, eine Phenyl-Gruppe, eine Phenylthio-Gruppe, die durch ein Halogen-Atom substituiert sein kann, oder eine Phenoxy-Gruppe, die durch wenigstens einen aus der aus einem Halogen-Atom, einer Trifluoromethyl-Gruppe, einer Nitro-Gruppe und $C_1$- bis $C_4$-Alkyl-Gruppen bestehenden Klasse ausgewählten Substituenten substituiert sein kann, bezeichnet und

n 1, 2 oder 3 bezeichnet, und, sofern n 2 oder 3 ist, die zwei oder drei durch X bezeichneten Substituenten gleich oder verschieden sein können und zwei Substituenten X zusammen mit den Kohlenstoff-Atomen in den Positionen 7 und 8 des Tetrahydrochinolin-Rings einen gesättigten oder ungesättigten Kohlenwasserstoff-Ring mit 5 bis 6 Kohlenstoff-Atomen bilden können,

mit der Massgabe, dass, wenn n 1 ist, X kein Chlor-Atom ist, und wenn n 2 oder 3 ist, alle X nicht gleichzeitig Chlor-Atome sind,
dadurch gekennzeichnet, dass

(a) Verbindungen der Formel (II)

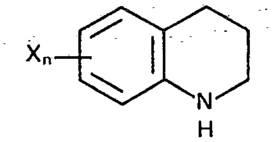

II

in der

X und n die angegebenen Bedeutungen haben, mit N,N'-Carbonyldiimidazol der Formel

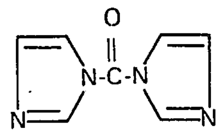

gegebenenfalls in Gegenwart inerter Lösungsmittel, umgesetzt werden oder

(b) Verbindungen der Formel (III)

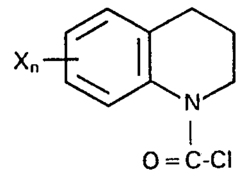

III

in der

X und n die angegebenen Bedeutungen haben, mit Imidazol der Formel

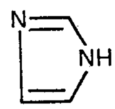

gegebenenfalls in Gegenwart von Säureacceptoren, umgesetzt werden.

5. Herbizide Mittel oder fungizide Mittel für Landwirtschaft und Gartenbau, dadurch gekennzeichnet, dass sie wenigstens ein Tetrahydrochinolin-1-ylcarbonylimidazol-Derivat der Formel (I) gemäss den Ansprüchen 1 bis 4 enthalten.

6. Verwendung von Tetrahydrochinolin-1-ylcarbonylimidazol-Derivaten der Formel (I) gemäss den Ansprüchen 1 bis 4 als Herbizide oder als Fungizide für Landwirtschaft und Gartenbau.

7. Verfahren zur Herstellung herbizider Mittel und fungizider Mittel für Landwirtschaft und Gartenbau, dadurch gekennzeichnet, dass Tetrahydrochinolin-1-ylcarbonylimidazol-Derivate der Formel (I) gemäss den Ansprüchen 1 bis 4 mit Streckmittel und/oder Trägern vermischt werden.

8. Verbindung der Formel

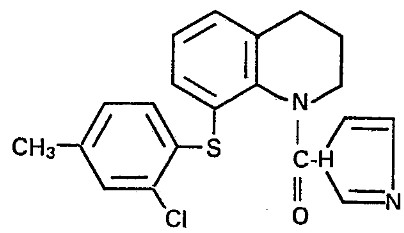

9. Verbindung der allgemeinen Formel (III)

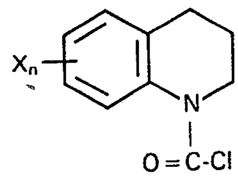

III

in der

X und n die in Anspruch 1 angegebenen Bedeutungen haben.

10. Verfahren zur Herstellung einer Verbindung der allgemeinen Formel (III)

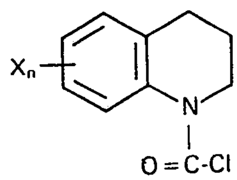

III

in der

X und n die in Anspruch 4 angegebenen Bedeutungen haben, dadurch gekennzeichnet, dass Verbindungen der allgemeinen Formel (II)

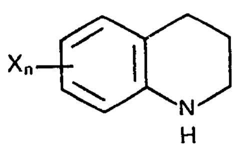

II

in der

X und n die im Vorstehenden angegebenen Bedeutungen haben, mit Phosgen oder Chlorameisensäure-trichloromethylester umgesetzt werden.

**Claims**

1. Tetrahydroquinolin-1-ylcarbonylimidazole derivatives of the general formula (I)

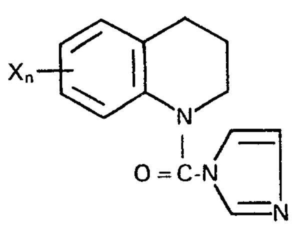

I

in which

X denotes a halogen atom, a $C_1$- to $C_4$-alkyl group, a nitro group, a cyano group, a trifluoromethyl group, a $C_1$- to $C_4$-alkoxy group which may be substituted by a halogen atom, a $C_1$- to $C_4$-alkylthio group which may be substituted by a halogen atom, a $C_1$- to $C_4$-alkylsulphonyl group, a phenyl group, a phenylthio group which may be substituted by a halogen atom, or a phenoxy group which may be substituted by at least one substituent selected from the class comprising a halogen atom, a trifluoromethyl group, a nitro group and $C_1$- to $C_4$-alkyl groups, and

n denotes 1, 2 or 3, and, if n is 2 or 3, the two or three substituents denoted by X may de identical or different and two substituents X, together with the carbon atoms in position 7 and 8 of the tetrahydroquinoline ring, can form a saturated or unsaturated hydrocarbon ring having 5 to 6 carbon atoms,

with the proviso that if n is 1, X is not a chlorine atom, and if n is 2 or 3, all X are not simultaneously chlorine atoms.

2. Compounds according to Claim 1, characterized in that

X denotes fluorine, chlorine, bromine, alkyl having 1 to 4 carbon atoms, trifluoromethyl or alkoxy having 1 to 4 carbon atoms which may be substituted by fluorine and/or chlorine,

n denotes 1 or 2, and if n is 2, the two X may be identical or different and two substituents X, together with the carbon atoms in positions 7 and 8 of the tetrahydroquinoline ring, can form a saturated or unsaturated hydrocarbon ring having 5 to 6 carbon atoms,

with the proviso that if n is 1, X is not chlorine, and if n is 2, the two X are not simultaneously chlorine.

3. Compounds according to Claim 1, characterized in that

X denotes fluorine, chlorine, methyl, ethyl, n-propyl, isopropyl, trifluoromethyl or methoxy, ethoxy, n-propoxy or isopropoxy which may be substituted by fluorine and/or chlorine, and denotes 2, and the two X may be identical or different and the two substituents X, together with the carbon atoms in positions 7 and 8 of the tetrahydroquinoline ring, can form a saturated or unsaturated hydrocarbon ring having 5 to 6 carbon atoms,

with the proviso that if n is 2, the two X are not simultaneously chlorine.

4. Process for the preparation of tetrahydroquinolin-1-ylcarbonylimidazole derivatives of the general formula (I)

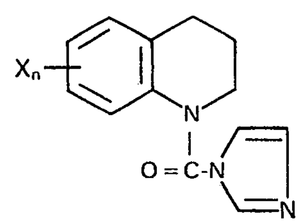

I

in which

X denotes a halogen atom, a $C_1$- to $C_4$-alkyl group, a nitro group, a cyano group, a trifluoromethyl group, a $C_1$- to $C_4$-alkoxy group which may be substituted by a halogen atom, a $C_1$- to $C_4$-alkylthio group which may be substituted by a halogen atom, a $C_1$- to $C_4$-alkylsulphonyl group, a phenyl group, a phenylthio group which may be substituted by a halogen atom,

or a phenoxy group which my be substituted by at least one substituent selected from the class comprising a halogen atom, a trifluoromethyl group, a nitro group and $C_1$- to $C_4$-alkyl groups, and

n denotes 1, 2 or 3, and, if n is 2 or 3, the two or three substituents denoted by X may be identical or different and two substituents X, together with the carbon atoms in positions 7 and 8 of the tetrahydroquinoline ring, can form a saturated or unsaturated hydrocarbon ring having 5 to 6 carbon atoms,

with the proviso that if n is 1, X is not a chlorine atom, and if n is 2 or 3, all X are not simultaneously chlorine atoms,
characterised that

(a) compounds of the formula (II)

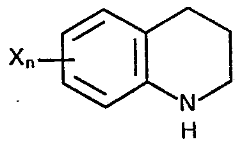

im which

X and n have the meanings specified, are reacted with N,N'-carbonyldiimidazole of the formula

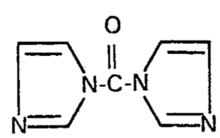

if appropriate in the presence of an inert solvent, or

(b) compounds of the formula (III)

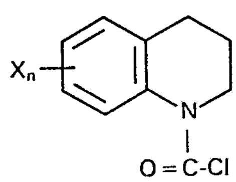

in which

X and n have the meanings specified, are reacted with imidazole of the formula

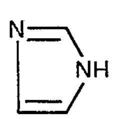

if appropriate in the presence of acid acceptors.

5. Herbicides or fungicides for agriculture and horticulture, characterized in that they contain at least one tetrahydroquinolin-1-ylcarbonylimidazole derivative of the formula (I) according to Claims 1 to 4.

6. Use of tetrahydroquinolin-1-ylcarbonylimidazole derivatives of the formula (I) according to Claims 1 to 4 as herbicides or fungicides for agriculture and horticulture.

7. Process for the production of herbicides and fungicides for agriculture and horticulture, characterized in that tetrahydroquinolin-1-yl-carbonylimidazole derivatives of the formula (I) according to Claims 1 to 4 are mixed with extenders and/or excipients.

8. Compound of the formula

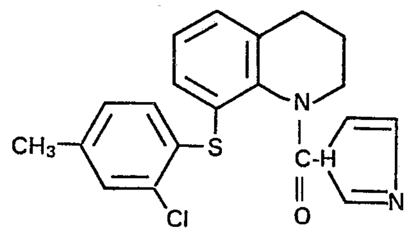

9. Compound of the general formula (III)

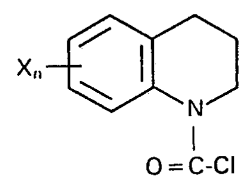

in which

X and n have the meaning specified in Claim 1.

10. Process for the preparation of a compound of the general formula (III)

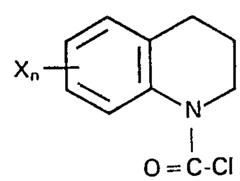 III

in which

X and n have the meanings specified in Claim 4, characterized in that compounds of the general formula (II)

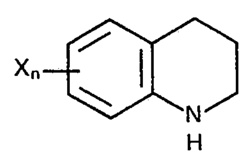 II

in which

X and n have the meanings specified above, are reacted with phosgene or trichloromethyl chloroformate.

## Revendications

1. Dérivés de tétrahydroquinoléine-1-ylcarbonylimidazole de formule générale (I)

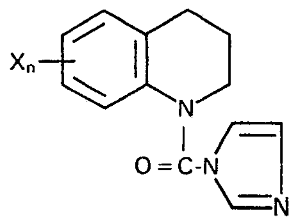

I

dans laquelle

X désigne un atome d'halogène, un groupe alkyle en $C_1$ à $C_4$, un groupe nitro, un groupe cyano, un groupe trifluorométhyle, un groupe alkoxy en $C_1$ à $C_4$ qui peut être substitué par un atome d'halogène, un groupe alkylthio en $C_1$ à $C_4$ qui peut être substitué par un atome d'halogène, un groupe alkylsulfonyle en $C_1$ à $C_4$, un groupe phényle, un groupe phénylthio qui peut être substitué par un atome d'halogène, un groupe phénoxy qui peut être substitué par au moins un substituant choisi dans la classe constituée d'un atome d'halogène, d'un groupe trifluorométhyle, d'un groupe nitro et d'un groupe alkyle en $C_1$ à $C_4$, et

n a la valeur 1, 2 ou 3, et, dans la mesure où n est égal à 2 ou 3, les deux ou trois substituants représentés par X peuvent être identiques ou différents, et deux substituants X peuvent former conjointement avec les atomes de carbone dans les positions 7 et 8 du noyau de tétrahydroquinoléine, un noyau hydrocarboné saturé ou non saturé ayant 5 ou 6 atomes de carbone,

sous réserve que, lorsque n est égal à 1, X ne représente pas un atome de chlore et que, lorsque n a la valeur 2 ou 3, tous les substituants X ne représentent pas en même temps des atomes de chlore.

2. Composés suivant la revendication 1, caractérisés en ce que

X représente le fluor, le chlore, et le brome, un groupe alkyle ayant 1 à 4 atomes de carbone, un groupe trifluorométhyle ou un groupe alkoxy ayant 1 à 4 atomes de carbone, qui peut être substitué par du fluor et/ou du chlore et

n a la valeur 1 ou 2 et, lorsque n est égal à 2, les deux substituants X peuvent être identiques ou différents, et deux substituants X peuvent former conjointement avec les atomes de carbone en positions 7 et 8 du noyau de tétrahydroquinoléine, un noyau hydrocarboné saturé ou insaturé de 5 ou 6 atomes de carbone,

sous réserve que, lorsque n est égal à 1, X ne soit pas du chlore et que, lorsque n est égal à 2, les deux substituants X ne représentent pas en même temps du chlore.

3. Composé suivant la revendication 1, caractérisé en ce que

X représente le fluor, le chlore, un groupe méthyle, éthyle, n-propyle, isopropyle, trifluorométhyle ou un groupe méthoxy, éthoxy, n-propoxy ou isopropoxy qui peuvent être substitués par du fluor et/ou du chlore, et

n a la valeur 2 et les deux X peuvent être identiques ou différents, et les deux substituants X peuvent former conjointement avec les atomes de carbone en positions 7 et 8 du noyau de tétrahydroquinoléine, un noyau hydrocarboné saturé ou non saturé de 5 ou 6 atomes de carbone,

sous réserve que, lorsque n est égal à 2, les deux X ne soient pas simultanément du chlore.

4. Procédé de production de dérivés de tétrahydroquinoléine-1-ylcarbonylimidazole de formule générale

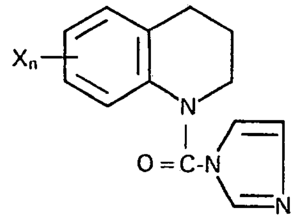

I

dans laquelle

X désigne un atome d'halogène, un groupe alkyle en $C_1$ à $C_4$, un groupe nitro, un groupe cyano, un groupe trifluorométhyle, un groupe alkoxy en $C_1$ à $C_4$ qui peut être substitué par un atome d'halogène, un groupe alkylthio en $C_1$ à $C_4$ qui peut être substitué par un atome d'halogène, un groupe alkylsulfonyle en $C_1$ à $C_4$, un groupe phényle, un groupe phénylthio qui peut être substitué par un atome d'halogène, un groupe phénoxy qui peut être substitué par au moins un substituant choisi dans la classe constituée d'un atome d'halogène, d'un groupe trifluorométhyle, d'un groupe nitro et d'un groupe alkyle en $C_1$ à $C_4$, et

n a la valeur 1, 2 ou 3, et, dans la mesure où n est égal à 2 ou 3, les deux ou trois substituants représentés par X peuvent être identiques ou différents, et deux substituants X peuvent former conjointement avec les atomes de carbone dans les positions 7 et 8 du noyau de tétrahydroquinoléine, un noyau hydrocarboné saturé ou non saturé ayant 5 ou 6 atomes de carbone,

sous réserve que, lorsque n est égal à 1, X ne représente pas un atome de chlore et que, lorsque n a la valeur 2 ou 3, tous les substituants X ne représentent pas en même temps des atomes de chlore,

caractérisé en ce que

(a) on fait réagir des composés de formule (II)

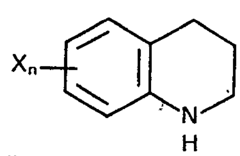

II

dans laquelle

X et n ont les définitions indiquées, avec le N,N'-carbonyldiimidazole de formule

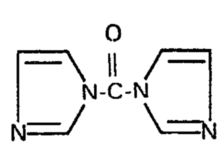

le cas échéant en présence de solvants inertes, ou bien

(b) on fait réagir des composés de formule (III)

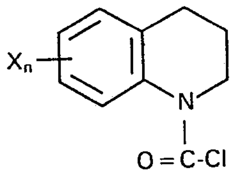

III

dans laquelle

X et n ont les définitions indiquées, avec l'imidazole de formule

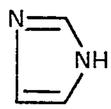

le cas échéant en présence d'accepteurs d'acides.

5. Compositions herbicides ou compositions fongicides pour l'agriculture et l'horticulture, caractérisées en ce qu'elles contiennent au moins un dérivé de tétrahydroquinoléine-1-ylcarbonylimidazole de formule (I) suivant les revendications 1 à 4.

6. Utilisation de dérivés de tétrahydroquinoléine-1-ylcarbonylimidazole de formule (I) suivant les revendications 1 à 4 comme herbicides ou comme fongicides pour l'agriculture et l'horticulture.

7. Procédé de préparation de compositions herbicides et de compositions fongicides pour l'agriculture et l'horticulture, caractérisé en ce qu'on mélange des dérivés de tétrahydroquinoléine-1-ylcarbonylimidazole de formule (I) suivant les revendications 1 à 4 avec des diluants et/ou des supports.

8. Composé de formule

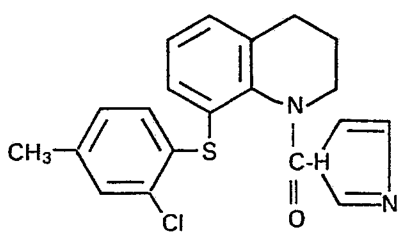

9. Composé de formule générale (III)

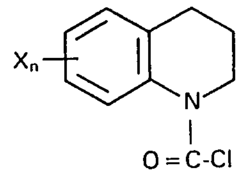

III

dans laquelle

X et n ont les définitions indiquées dans la revendication 1.

10. Procédé de préparation d'un composé de formule générale (III)

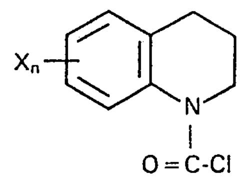

III

dans laquelle

X et n ont les définitions indiquées dans la revendication 4,

caractérisé en ce qu'on fait réagir des composés de formule générale (II)

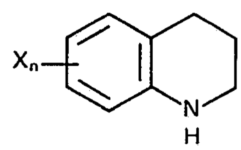

II

dans laquelle

X et n ont les définitions indiquées ci-dessus, avec le phosgène ou l'ester trichlorométhylique de l'acide chloroformique.